# EUROPEAN PATENT APPLICATION

(11) **EP 1 929 862 A1**
(43) Date of publication of application: **11.06.2008**
(21) Application number: 08151955.5
(22) Date of filing: 18.08.2003
(51) Int. Cl.: A01N 1/00

(54) **Allograft tissue purification process for cleaning bone**

(30) Priority: 23.08.2002 US 226214
(62) Divisional of application: 03792993.2
(71) Applicant: Musculoskeletal Transplant Foundation, Edison, New Jersey 08837 (US)
(72) Inventor: The designation of the inventor has not yet been filed
(74) Representative: W.P. Thompson & Co.

(57) **Abstract**

A method for producing a cleaned bone graft with osteoinductivity above 2.0 suitable for transplantation into a human. The first step is sonicating a bone graft in a nonionic detergent in an ultrasonic cleaner at a temperature ranging from about 33°C to about 37°C and for a time period ranging from 15 minutes to 2 hours effective to produce a cleaned bone graft essentially free from bone marrow. The bone graft is sonicated in purified water in an ultrasonic cleaner at a temperature ranging from about 33°C to about 37°C a plurality of times to remove the detergent producing a cleaned bone graft. The bone graft is then sonicated in hydrogen peroxide in an ultrasonic cleaner at a temperature ranging from about 33°C to about 37°C for a time period ranging from 10 minutes to about 2 hours effective to retain osteoinductivity of the bone graft and again sonicated in purified water in an ultrasonic cleaner at a temperature ranging from about 33°C to about 37°C a plurality of times to produce a cleaned bone graft. The final step is sonicating the bone graft in an alcohol at a temperature ranging from 33°C to 37°C for 30 minutes to 2 hours, all of the steps being effective to reduce any initially present viruses at least two logs and bacteria at least ten logs and the bone graft is finally sonicated in purified water to remove the alcohol.

## Description

### RELATED APPLICATIONS

There are no related applications.

### FIELD OF THE INVENTION

The present invention generally relates to a method for cleaning bones to produce bone grafts suitable for transplantation into a human. The process more specifically involves the use of ultrasonic cleaning in the removal of bone marrow, tissue, bacteria and viruses by causing ultrasonic cavitation in sequential detergent, hydrogen peroxide and alcohol solutions.

### BACKGROUND OF THE INVENTION

One hundred to two hundred thousand tissue transplants are annually performed in the United States. The single most variable factor with respect to allographic transplantation is the preparation of such bone and tissue segments. Procedure and protocol of the some 400 tissue banks in North America are quite varied and has resulted in various technology with developed processes.

Allografts are vital for bone stock deficiencies that occur during orthopaedic trauma, joint reconstruction, or other reconstructive procedures. The main criteria for an orthopaedic allograft are the retention of strength, the retention of biologic factors, and the reduction of risk of disease transmission. The first two should not be affected by processing, while processing should eliminate the risk for disease transmission.

There is no known industry standard specifying levels of cleanliness for cleaning and preparing bone segments. The problems associated with this lack of standards interpret to poor process control, inadequate removal of tissue from the parent surface and to a large extent lack of sterility during the tissue recovery process.

Human bone obtained from cadaveric donors is typically procured under sterile conditions in an operating suite environment of local hospitals. The bone is stored frozen until it is further processed into small grafts under similar sterile conditions, or under clean-room conditions. Procurement and processing of human tissues is typically performed by groups certified by the American Association ofTissue Banks under standard operating procedures for the processing of each specific bone graft. Large bones such as the femur are thawed and debrided ofexcess tissue prior to being cut into smaller grafts. Processing ofthe smaller grafts includes cleaning ofbone marrow from the cancellous bone spaces. Cleaning of bone marrow and tissue from small bone grafts has been described in the scientific literature and in brochures and documents made public by groups involved in the procurement and processing of human tissues.

Osteotech, Inc. describes a bone graft cleaning process called Permein.TM. ("a combination of ethanol and non-ionic detergent" which involves the use of a solution of ethanol and detergent to clean bone grafts.

Detergents are amphophil compounds which facilitate solubilization of relatively insoluble lipids present in, for example, bone marrow, yet at higher concentrations tend to form micellar structures (Helenius, A. and Simons, K. Solubilization of Membranes by Detergents, Biochim. Biophys. Acta 415 (1975) 29-79). The formation of micellar structures tend to limit the effective concentration range for detergent solutions and thus soaking of bone in a given volume of detergent solution may not be totally effective in that the absolute amount of detergent present is limited and if the amount oflipid material to be solubilized exceeds the solubilization capability of the detergent present, lipid solubilization will not be complete.

The use of prior art procedures to remove bone marrow involves the use of pressurized flow of solution as a rapidly moving stream which dislodges bone marrow by impact of the solvent on the bone graft. Such procedures tend to generate aerosols oftissue and solvent which can be hazardous to processing personnel. The present invention virtually eliminates this hazard.

Ultrasonic cleaners are extensively used in cleaning glass tubes, metal instruments, filters, etc. Ultrasound is sound transmitted at frequencies beyond the range of human hearing. Ultrasonic energy in liquid generated by piezoelectric or other types of transducers creates cavitation, which is the mechanism for ultrasonic cleaning. Cavitation consists of the formation and collapse of countless tiny cavities, or vacuum bubbles, in the liquid. The energy produces alternating high and low pressure waves within the liquid of a tank. The liquid is compressed during the high pressure phase of the wave cycle, then pulled apart during the low pressure phase. As the pressure in the liquid is reduced during the low pressure phase, cavities grow from microscopic nuclei to a maximum critical diameter. During the subsequent high pressure phase they are compressed and implode. The energy is powerful, but safe for parts because it is localized at the microscopic, i.e., cellular, scale. Factors affecting the strength of cavitation are temperature, surface tension, detergents or other agents which reduce surface tension are optimal, viscosity (medium vapor pressure is most conducive to ultrasound activity), and density (where high density creates intense cavitation with greater implosive force).

A number of prior art references have used ultrasonics together with detergents and other solutions to clean bone.

In U.S. Patent Numbers 5,556,379 issued September 17, 1996 and 5,976,104 issued November 2, 1999, it is noted that processing of the smaller grafts includes cleaning of bone marrow from the cancellous bone spaces using mechanical means, soaking, sonication, and/or lavage with pulsatile water flow under pressure. This cleaning may use reduced or elevated temperatures, for example 4º C to 65º C, and may also include the use of detergents, alcohol, organic solvents or similar solutes or combination of solutes designed to facilitate solubilization of the bone marrow.

In the Simonds reference from the New England Journal of Medicine, page 726, March 12,1992, entitled TRANSMISSION OF HUMAN IMMUNODEFICIENCY VIRUS TYPE I FROM SERONEGATIVE ORGAN AND TISSUE DONOR, the bone was lyophilized and treated with ethanol. (Step 1 or 3). The lyophilized tissue had soft tissue removed, followed by treatment with two antibiotics (Step 2), irrigation with sterile water, packaging and refreezing and lyophilization to a residual moisture content of less than 5%. The ethanol treated tissue underwent ultrasonic cleaning in 30% ethanol (Step 1), removal of marrow by water lavage and brief treatment in 100% ethanol (Step 3).

Another pertinent reference is the article A Review of Allograft Processing and Sterilization Techniques and Their Role in Transmission of the Human Immunodeficiency Virus, American Journal of Sports Medicine, Vol21, No. 2 ((1993) presented at the interim meeting of AOSSM, February 1993, San Francisco California (Exhibit 6) sponsored by LifeNet Transplant Services.

The article states in part: "Bone preserved by freeze-drying was first thawed, and followed by removal of extraneous soft tissue. It was then placed in the ultrasonic cleanser in 30% ethanol solution. All marrow elements were removed by pulsating water lavage, followed by 15 minutes in 100% ethanol and brief irrigation with bacitracin and polymyxin B sulfate solution. These tissues were then washed and packaged in glass containers and refrozen."

U.S. Patent Number 5,095,925 issued March 17,1992 is directed toward a bone cleaning device using ultrasonics which removes gross tissue from bone to prepare the same for transplant and use in surgery. The bone is subjected to a positive pressure stream of sterile water, ultrasonically cleaning the same in a detergent followed by rinsing and soaking and reintroduced to the ultrasonic process if necessary within a preferred working temperature range of 27º C to 33º C.

U.S. Patent Number 5,509,968 issued April 26, 1996 is directed toward cleaning used orthopaedic implants which are decontaminated and made available for reuse by a three step process for removal of protein tissue, bone tissue and lipids.

The '968 patent uses a process to clean then implant in the following manner: The implant is suspended in an aqueous bath of detergent suitable for emulsifying lipids at elevated temperatures, such as 40º C to 60º C, and is typically treated for about 1 to 45 minutes by the use of an ultrasonic cleaning system. The solution in the treating container is discarded and the container and implant are washed with clean water. A container is filled with a dilute acid capable of dissolving bone salts (e.g., calcium phosphate minerals that are deposited in the collagen matrix of the bone). The implant is added to the container, and subjected to ultrasonic treatment for approximately the same time. After treatment, the solution containing dissolved bone salts is discarded and the implant and container are again rinsed with clean water. The implant is then subjected to a bath of an aqueous solution sodium hypochlorite of a concentration as sold for general cleaning purposes, (household bleach). This step removes any remaining organic bone tissue as well as protein. An ultrasonic cleaning system is again used for the same time and temperature. When this step is completed, the solution is discarded and both the implant and container rinsed with water.

In U.S. PatentNumber 5,333,626 issued August 2,1994, a high pressure wash is used to clean bone. The bone is cleaned with a high pressure detergent solution such as TritonX-100 and Tween 80 preferably from 37º C to 80º C. Following the washing, the solution is changed. Sterile water or biologically acceptable alcohol is used to remove the detergent and it is removed by rinsing with sterile water. The bone may be further decontaminated by exposing it to 3% hydrogen peroxide solution from 5 to 120 minutes (preferably 5 to 60 minutes) after which the residual hydrogen peroxide is removed by washing with sterile water. After cleaning, the bone is finally decontaminated by contacting the bone with a global decontaminate for 30 to 60 minutes. U.S. Patent Number 5,797,871 issued August 25,1998 is also directed toward a bone cleaning pro cess using ultrasonics in which the bone is sonicated in a solution of several detergents within a temperature range of 37º C to 50º C to produce bone grafts essentially free from bone marrow and detectable fungal and viral contamination.

### SUMMARY OF THE INVENTION

The present invention is directed toward a process for cleaning bones to remove tissue, bone marrow, bacteria, fungi and viruses by sonicating the bone in anonionic detergent solution, draining the detergent solution and respectively soaking and sonicating the bone in a purified water bath to remove the detergent, draining the water bath and adding a hydrogen peroxide solution in which the bone is sonicated. The hydrogen peroxide is drained and the bone is soaked and sonicated in purified water to remove hydrogen peroxide and soaking and sonicating the bone in an alcohol solution, draining the same and soaking and sonicating the bone in purified water to remove the alcohol. The process arrives at a bone which has a viral clearance of at least two logs and retains its osteoinductivity.

An object ofthe present invention is to provide a means of removing bone marrow from the luminal and cancellous bone spaces in essentially intact bone grafts and small machined, shaped bone grafts.

It is a further object of the invention to process essentially intact bone grafts with minimal residual bone marrow as bone marrow may harbor potential viral particles and/or viral genomes integrated into the genomes of specific cell types present in the bone marrow, thus reducing the potential for transmission of infective agents such as bacteria and viruses.

Another object of this invention is to use detergent and hydrogen peroxide solutions in the processing of bone grafts. Hydrogen peroxide and detergents have been demonstrated to be virucidal towards viruses such as HIV and hepatitis and certain bacteria, and to enhance cavitation associated with ultrasonic cleaners. Alcohol and detergent solutions also offer advantages of enhancing solubilization of bone marrow, reducing surface tension properties of aqueous solutions, and inactivating viruses and bacteria.

Still another object is to use ultrasonic cleaners which offer advantages of cavitation events which facilitate disruption and breakdown of soft tissues at the microscopic level.

It is yet another object ofthe invention that the present invention has particular utilization in the medical field where the article being cleaned is a surgical device for allograft, autograft, xenograft and artificial transplants.

It is a particular object of this invention to provide a system for the sterile preparation of transplantable tissue. The needs filled by said system on a broad scale will be a unification of procedure and protocol for allograft tissue preparation, consistent performance and results of tissue processing centers, reduced detrimental effects oftoxic chemicals and radiation now used by several processors in the bone banking community, increase in the quality of allograft materials produced, decreased post-operative infection and transmission of disease, negation of local environmental factors such as toxic and pollutants and quicker functional incorporation of transplanted allografts.

It is another object of the present invention to provide a system for the sterile preparation of transplantable tissue that will avoid the application of secondary sterilants that produce deleterious effects in the host site and body.

It is yet another object of the present invention to provide a system for the sterile preparation of transplantable tissue that will increase the shelf life, product quality, and component integrity, derive consistent and uniform results of prepared tissue and reduce the preparatory expense.

These and other objects, advantages, and novel features of the present invention will become apparent when considered with the teachings contained in the detailed disclosure which along with the accompanying drawings constitute a part of this specification and illustrate embodiments ofthe invention which together with the description serve to explain the principles of the invention.

### BRIEF DESCRIPTION OF THE DRAWINGS

Figure 1 illustrates a flow chart diagram of a preferred embodiment of the present method for producing a cleaned bone graft; and

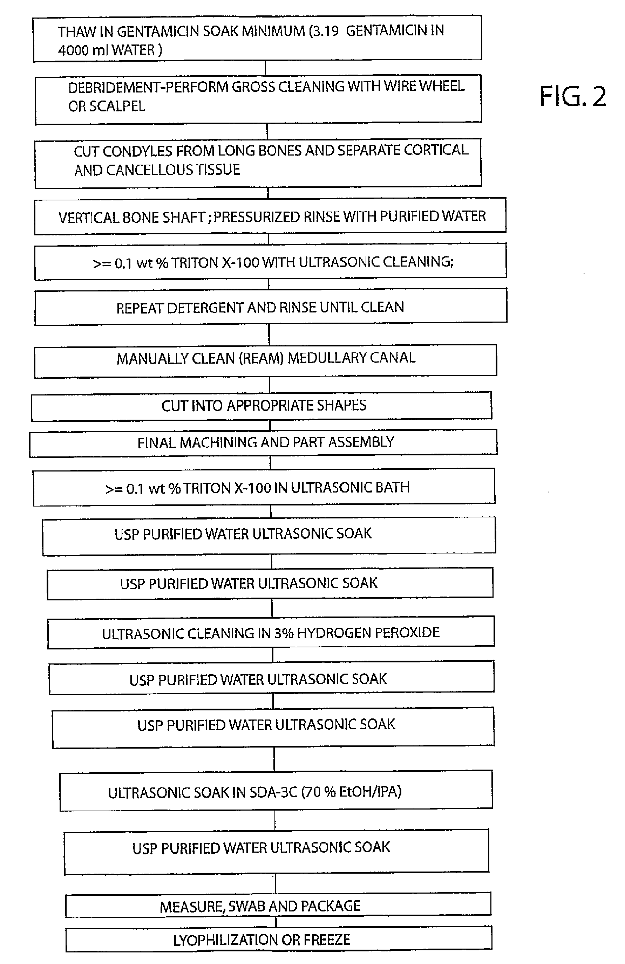

Figure 2 illustrates a flow chart diagram of an alternative embodiment of the present method for producing a cleaned bone graft;

### DETAILED DESCRIPTION OF THE INVENTION

### I. Definitions.

The below definitions serve to provide a clear and consistent understanding of the specifications and claims, including the scope to be given such terms.

Bone Graft. The term "Bone Graft" is intended any bone obtained from a cadaver donor, for example any shaped bone part and/or any small cut pieces of bone.

Cleaning Container. By the term "cleaning container" is intended for the purpose of the present invention any container of a size sufficient to contain the bone graft beingprocessed. The cleaning container used was a 4 liter stainless steel container with wire mesh to support the bone graft.

Detergent. By the term "detergent" is intended any agent which through a surface action that depends on it possessing both hydrophilic and hydrophobic properties and/or exerts oil-dissolving (cleansing) and/or antibacterial and/or antiviral effects.

Ultrasonic Cleaner. The term "ultrasonic cleaner" is intended any ultrasonic cleaning device capable of operating from 20 kHz to 1000 kHz, preferably at about 40 kHz with an energy level ranging from 10-180W/sq.in., and includes, for example, but not by way of limitation, a Branson 8000 Ultrasonic Cleaner Model Numbers: 84540-18 or any similar ultrasonic cleaner.

Bone Marrow. The term "bone marrow" is intended for the purposes of the present invention the highly cellular hematopoietic connective tissue filling the medullary cavities and spongy epiphyses of bones which may harbor bacterial and/or viral particles and/or fungal particles.

Solvent. The term "solvent" is intended for the purposes ofthe present invention, a liquid cleaning composition capable of: facilitating the solubilization of lipid, facilitating bone marrow removal, inactivating viral and/or bacterial particles, and/or disrupting cell membranes, which may contain, but is not limited to, one or more ofthe following: sterile water; saline; a detergent; an alcohol, for example, ethanol and/or isopropanol or a combination of same, solvents, a combination of solutes desired to facilitate solubilization ofbone marrow, for example, chelating agent; virucidal agent; bacteriocidal agent; antimycotic agent; sodium hydroxide or similar strong base, organic and/or inorganic acid and hydrogen peroxide.

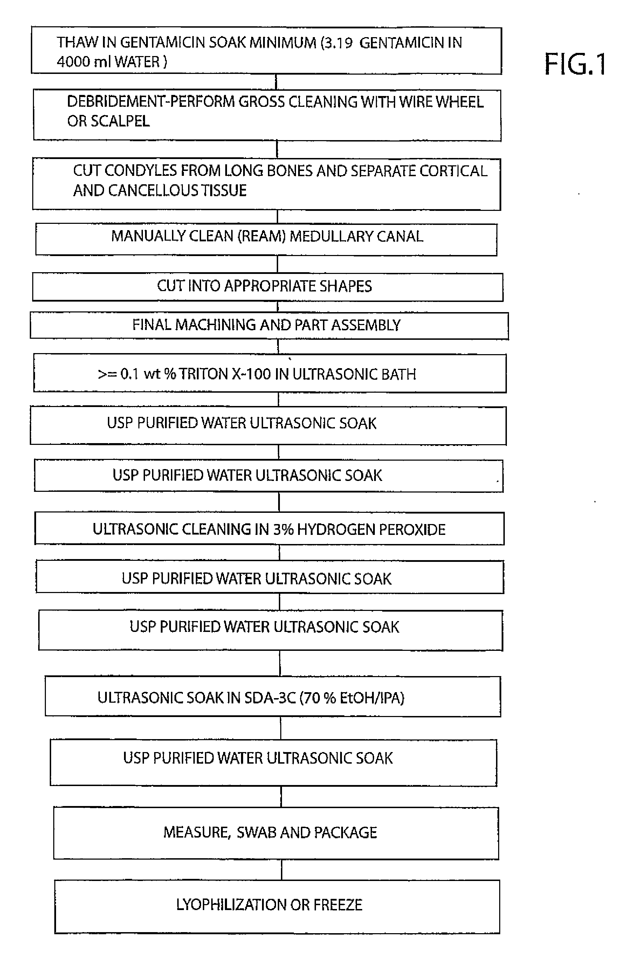

While the present invention and best mode ofthe invention is shown in Figure 1 and will be described in connection with certain preferred embodiments, it is not intended that the present invention be so limited. On the contrary, it is intended to cover all alternatives, modifications, and equivalent arrangements as may be included within the spirit and scope of the invention as defined by the appended claims.

The present invention is directed toward the cleaning and processing of bone grafts using a nonionic detergent soak (see Table 1), a hydrogen peroxide soak, and 70% alcohol (ethanol and isopropanol) soak, and frequent intermittent purified water washes, all under temperature controlled sonication. In the process, bone graft samples were processed as control (0-hour H₂O₂ treatment, with no sonication) or treatment (5-hour H₂O₂ treatment). Hydrogen peroxide (H₂O₂) is an oxidizing chemical used to process bone allografts with the potential to eradicate microorganisms and viruses. It was previously thought that hydrogen peroxide could potentially compromise osteoinductivity and bone structural proteins.

Compression cylinders (5.3-mmx 5.3-mm) of the bone grafts were fabricated from human femurs (age 39M & 61F) oriented longitudinally and transversely, and were preserved both frozen (-70º C) and freeze-dried (N=8 for all groups). Freeze dried samples were rehydrated for at least 1-hour prior to testing and frozen samples were soaked for at least 15-minutes prior to testing, both in normal saline. Samples were loaded to failure in uniaxial compression at a strain rate of 0-01 per second and maximum and yield stress were calculated. Impact specimens were fabricated into anterior cervical fusion (ACF) allografts from fibulas (age 46M, 21M, 60M, & 62M), and were preserved both frozen and freeze-dried (N=5 for all groups). Samples were secured into a custom fixture using 3 N-m of torque and impacted starting at 5-cm with 1-cm increments, using an ACF impact tool, and an 841-g carriage, until failure. Total kinetic energy at failure was calculated for each ACF.

**Osteoinductivity:** Cortical bone from three donors (37M, 49M, 58F) was processed using a control process, 0 hour H₂O₂ with no sonication, a treatment process with 1 hour, 3 hours, and 5 hours of H₂O₂ treatment, and a negative control. After processing, the bone was ground, demineralized, and prepared into 32% Demineralized Bone Matrix in a hyaluronan carrier. Samples (15 mg) were implanted bilaterally into the hamstring muscle in an athymic mouse model, approved by the University of Medicine and Dentistry of New Jersey animal care and use committee. Implants were evaluated histologically after 28 days.

**Viral Clearance:** Cortical bone samples were processed with a 1-hour H₂O₂ step. Samples were subjected to the previous steps of the process. For each of six representative viruses, for each step of the process, samples were spiked with a virus suspension, and subjected to the given treatment step, while a control was subjected to an inert, zero-time, but equal-volume version of the same step. Supernatant was recovered from these samples, neutralized (where appropriate), and assayed for viral activity, using plaque and similar assays. Viral reduction for each virus for each step was calculated as the difference between the viral titer of the control, and the viral titer of the test sample at the full cycle time for the given treatment step-Results are the sum of the log reductions for all treatment steps for each virus.

**Bacteriological Sterility:** Cortical bone samples were subjected to the previous steps of the process. For each of six representative bacteria, for each step ofthe process, sample surfaces were treated at designated protocol bacteria concentration and subjected to a one quarter time increment of the preferred treatment step and a full time increment of the preferred treatment, while a control was subjected to an inert, zero-time, but equal-volume version of preferred treatment step. The samples were assayed for bacteria activity, using standard assays. Bacteria reduction for each ofthe bacteria was calculated as the difference between the organism titer of the control; and the organism titer of the test sample at one quarter cycle time (t₁ Chart 2) and the full cycle time (t₂ Chart 2) for the given treatment step. Results are the sum of the log reductions for all treatment steps for each bacteria.

### RESULTS IN TESTING

**Mechanical:** The results of the compression testing show no significant differences between the control and treatment group maximum stress data (Table 2). Results of the impact testing revealed no significant differences between the control and treatment groups (Table not shown; the means (standard deviation) are: control = 49.8 (45.7); treatment = 35.2 (22.6)).

**Osteoinductivity:** Hydrogen peroxide cleaning had a statistically significant effect on osteoinductivity, giving a linear decrease with increasing peroxide time (Chart 1). The mean (SD) osteoinductivity scores were 3.65 (0.49) for 0 hours, 3.04 (0.97) for 1 hour, 2.57 (1.36) for 3 hours, 1.47 (1.10) for 5 hours H₂O₂ treatment times. The negative control score was zero. Compared to the control (0 h), the 1 hour score was not significantly different (p=0.113), and the 3 hour and the 5 hour scores were significantly different (p=0.045 & p=0.0001, respectively).

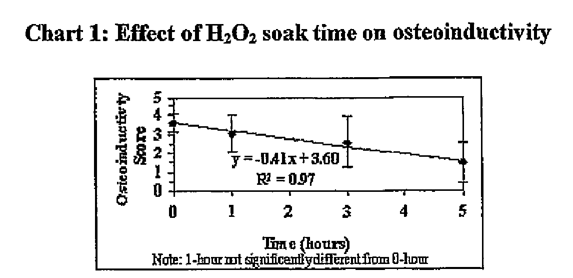

Under current osteoinductivity standards, a score of 4.0 to 3.0 is highly osteoinductive, 3.0 to 2.0 is moderately osteoinductive, 2.0 to 1.0 is slightly osteoinductive.

**Viral Clearance:** The results of the viral clearance study demonstrate that processing the cortical bone allografts in a nonionic detergent, H₂O₂, and alcohol gives viral reductions greater than six logs in all cases except the PPV virus (Table 3). Under FDA definitions, viruses can be listed as cleared at two logs or better. It should be specifically noted that in the present invention, HIV virus is reduced one quadrillion times (10¹⁵).

Processing the bone graft with a 5-hour H₂O₂ soak does not affect the compression strength of cortical bone allografts. Likewise, the impact data did not show any statistical differences. The osteoinductivity score for the 1 -hour H₂O₂ treatment time is favorable, because no significant statistical decrease was seen. The 3 hours and 5 hours treatment times were undesirable, as they caused statistically significant decreases in osteoinductivity. The viral clearance result verifies that the risk for disease transmission can be greatly reduced or eliminated by processing, beyond standard donor testing and screening procedures.

**Bacteriological Sterility:** The results ofthe bacteriological sterility study demonstrate that processing the cortical bone allografts in a nonionic detergent, H₂O₂, and alcohol soaks gives bacteria reductions greater than ten logs. Under FDA definitions, bacteriological reduction for a single step is considered effective at two logs or better. It should be noted that one bacteria Clostridium Sporogenes was not tested under an acceptable protocol and thus is not listed in Chart 2 below.

The bacteria Candida albicans, Staphylococcus aureus, Staphylococcus epidermidis, Escherichia coli, Pseudomonas aeruginosa, and Bacillus subtilis were all reduced after complete treatment at least ten logs. The bacteriological sterility result verifies that the risk for disease transmission from bacteria can be greatly reduced or eliminated by processing, beyond standard donor testing and screening procedures.

### Chart 2: Microbiological Reduction Results

| Microbiological Log Reductions | | | | | |
|---|---|---|---|---|---|
| Organism | Process Step | T₁ | T₂ | T₁ Total | T₂ Total |
| *Candida albicans* | Triton X-100 | 1.78 | 1.58 | >11.11 | >10.74 |
| | H₂O₂ | >5.47 | >5.10 | | |
| | Alcohol | >5.64 | >5.64 | | |
| *Staphylococcus aureus* | Triton X-100 | -0.59 | 0.20 | >11.78 | >11.78 |
| | H₂O₂ | >5.93 | >5.93 | | |
| | Alcohol | >5.85 | 5.85 | | |
| *Staphylocaccus epidermidis Staphylococcus epidermidis* | Triton X-100 | 0.56 | 0.41 | >10.82 | >10.82 |
| | H₂O₂ | >5.21 | >5.21 | | |
| | Alcohol | >5.61 | >5.61 | | |
| *Escherichia coli* | Triton X-100 | 0.03 | 1.82 | >10.44 | >10.44 |
| | H₂O₂ | >5.19 | >5.19 | | |
| | Alcohol | >5.25 | >5.25 | | |
| *Pseudomonas aeruginosa* | Triton X-100 | 2.48 | 3.40 | >13.70 | >13.70 |
| | H₂O₂ | >5.09 | >5.09 | | |
| | Alcohol | >5.21 | >5.21 | | |
| *Bacillus subtilis* (vegetative) | Triton X-100 | 2.56 | 2.41 | >11.11 | >11.28 |
| | H₂O₂ | >5.25 | >5.25 | | |
| | Alcohol | 3.30 | 3.62 | | |

Overall, these results demonstrate that it is possible to clean cortical bone allografts without causing a reduction in mechanical strength or a significant loss in osteoinductivity, while at the same time significantly reducing the risks of disease transmission.

**Table 1: List of Non-ionic Detergents**

| |
|---|
| N, N-Dimethyldodecylamino-N-oxide |
| Octylglucoside |
| Polyoxyethylene (PEG) alcohols |
| Polyoxyethylene-p-t-octylphenol |
| Polyoxyethylene nonylphenol |
| Polyoxyethylene sorbitol esters |
| Polyoxy-propylene-polyoxyethylene esters |
| p-isoOctylpolyoxy-ethylene-phenol formaldehyde polymer |

**Table 2: Compression maximum stress data (MPa) comparing the control groups to the 5-hour H₂O₂ treatment test groups**

| Storage | Donor Info | Tissue Orientation | | | | | |
|---|---|---|---|---|---|---|---|
| | | Longitudinal | | | Transverse | | |
| | | Control | Test | Pr(F) | Control | Test | Pr(F) |
| Frozen | 39m | 164 (7) | 159 (9) | 0.25 | 128 (9) | 119 (10) | 0.07 |
| | 61f | 156 (5) | 159 (8) | 0.42 | 124 (11) | 121 (9) | 0.52 |
| Freeze-Dried | 39m | 219 (27) | 222 (27) | 0.82 | 153 (20) | 167 (25) | 0.24 |
| | 61f | 206 (27) | 202 (38) | 0.82 | 127 (15) | 117 (15) | 0.21 |

| | | | | | | | |
|---|---|---|---|---|---|---|---|
| Means are presented with their corresponding standard deviations in parentheses. For all groups, N = 8. The probability that the means are equal is given as Pr(F). Values < 0.05 are considered statistically different. | | | | | | | |

**Table 3: Total viral clearance in cortical bone allograft due to processing in detergent, 1-hour hydrogen peroxide, and alcohol**

| Virus | RNA or DNA | Model for | Total Log Reduction of Virus |
|---|---|---|---|
| Enveloped Viruses | | | |
| *BVDV (bovine viral diarrhea)* | RNA | Hepatitis C (HCV) | >10.62 |
| *HIV (human immunodeficiency)* | RNA | HIV | >15.22 |
| *PrV (Pseudorabies)* | DNA | CMV/Herpes | >12.23 |

| Non-Enveloped Viruses | | | |
|---|---|---|---|
| *HAV (Hepatitis A)* | RNA | HAV | >6.46 |
| *Polio* | RNA | Polio/picornaviridae | >10.96 |
| *PPV (porcine parvovirus)* | DNA | Human parvovirus B19 | 2.57 |

### Process 1:

In the allograft tissue purification process, cortical bones are taken from the normal bone recovery process where they have been frozen and shipped or stored for processing. The bone tissue was thawed in a Gentamicin soak (3.1g Gentamicin in 4000 ml water) for 15 minutes to 2 hours. A debridement process was performed, gross cleaning the bone sample with a wire wheel or scalpel for 30 minutes. Condyles were cut from the long bones and cortical and cancelleous tissue was separated. Processing was begun on the cortical tissue whereby the medullary canal was manually cleaned for 30 minutes. The cortical tissue was cut in appropriate rough bone part shapes, a step which lasted anywhere from 30 minutes to 3 hours, depending on the donor and bone part shape. After rough cutting, the next step involved final machining and part assembly of the control tissue, taking anywhere from 5 minutes to 5 hours depending on the bone part being machined.

Each bone part was then subjected to an ultrasonic bath of a nonionic detergent consisting of greater than or about 0.1 wt.% Triton X-100 for 30 minutes at 34º C, plus or minus 1º C. This ultrasonic bath can also use Tween 80 or if desired, another nonionic detergent such as N, N-Dimethyldodecylamino-N-oxide, Octylglucoside, Polyoxyethylene (PEG) alcohols, Polyoxyethylene-p-t-octylphenol, Polyoxyethylene nonylphenol, Polyoxyethylene sorbitol esters, Polyoxy-propylene-polyoxyethylene esters, p-isoOctylpolyoxy-ethylene-phenol formaldehyde polymer can be used. An ionic detergent will degrade proteins and effect o steoinductivity ofthe bone graft. The detergent was drained and the bone graft part was subjected to a 5 minute ultrasonic soak of USP purified water at 34º C, plus or minus 1º C. The water soak was emptied and repeated, totaling 2 separate soaks to remove the detergent and a final water soak was instituted comprising a 30 minute continuous ultrasonic soak in USP purified water at 34º C, plus or minus 1º C. After the final water soak was emptied, the cortical bone part sample was ultrasonically cleaned in 3% hydrogen peroxide at 34º C, plus or minus 1º C for 15 minutes to 2 hours, preferably 1 hour. The hydrogen peroxide concentration can range from 1.5% to 30% depending on the time and temperature used. After emptying the hydrogen peroxide, the bone part is again subjected to a 5 minute ultrasonic soak of USP purified water at 34º C, plus or minus 1º C. The soak was emptied and repeated for a total of 2 soaks, drained and followed with a 30 minute continuous ultrasonic soak in USP purified water at 34º C, plus or minus 1º C. After the purified water soak is emptied, the cortical bone sample part was ultrasonically soaked in an alcohol solution SDA-3C (70% EtOH/IPA) at 34º C, plus or minus 1º C for 30 minutes to 2 hours, preferably 1 hour, drained and followed by another ultrasonic soak in USP purified water at 34º C, plus or minus 1º C for 5 minutes. The purified water soak was emptied and repeated a total of 2 soaks to remove the alcohol and the sample part removed. The sample part was then measured, swabbed and packaged. The package containing the sample undergoes lyophilization or freezing and then undergoes quality assurance.

### Process 2:

In the allograft tissue purification process, cortical bones are taken from the normal bone recovery process where they have been frozen and shipped or stored for processing. The bone tissue was thawed in a Gentamicin soak (3.1 g Gentamicin in 4000 ml water) for 15 minutes to 2 hours. A debridement process was performed, gross cleaning the bone sample with a wire wheel or scalpel for 30 minutes. Condyles were cut from the long bones and cortical and cancelleous tissue was separated. Processing was begun on the cortical tissue whereby the medullary canal was manually cleaned for 30 minutes. The cortical tissue was cut in appropriate rough bone part shapes, a step which lasted anywhere from 30 minutes to 3 hours, depending on the donor and bone part shape. After rough cutting, the next step involved final machining and part assembly of the control tissue, taking anywhere from 5 minutes to 5 hours depending on the bone part being machined.

The vertical bone shaft is subjected to ultrasonic cleaning in 0.1 wt.% Triton X-100 for 1 to 3 hours at 34ºC, plus or minus 1º C. The vertical bone shaft is subjected to a pressurized rinse with purified water for up to 1 hour and drained. This cleaning detergent bath of 0.1 wt.% Triton X-100 and rinse is repeated with sonication until clean.

Each bone part was then subjected to an ultrasonic bath of a nonionic detergent consisting of greater than or about 0.1 wt.% Triton X-100 for 30 minutes at 34º C, plus or minus 1º C. This ultrasonic bath can also use Tween 80 or if desired, another nonionic detergent such as N, N-Dimethyldodecylamino-N-oxide, Octylglucoside, Polyoxyethylene (PEG) alcohols, Polyoxyethylene-p-t-octylphenol, Polyoxyethylene nonylphenol, Polyoxyethylene sorbitol esters, Polyoxy-propylene-polyoxyethylene esters, p-isoOetylpolyoxy-ethylene-phenol formaldehyde polymer can be used. Anionic detergent will degrade proteins and effect osteoinductivity of the bone graft. The detergent was drained and the bone graft part was subjected to a 5 minute ultrasonic soak of USP purified water at 34º C, plus or minus 1º C. The water soak was emptied and repeated, totaling 2 separate soaks to remove the detergent and a final water soak was instituted comprising a 30 minute continuous ultrasonic soak in USP purified water at 34º C, plus or minus 1º C. After the final water soak was emptied, the cortical bone part sample was ultrasonically cleaned in 3% hydrogen peroxide at 34º C, plus or minus 1º C for 15 minutes to 2 hours, preferably 1 hour. The hydrogen peroxide concentration can range from 1.5% to 30% depending on the time and temperature used. After emptying the hydrogen peroxide, the bone part is again subjected to a 5 minute ultrasonic soak of USP purified water at 34º C, plus or minus 1º C. The soak was emptied and repeated for a total of 2 soaks, drained and followed with a 30 minute continuous ultrasonic soak in USP purified water at 34º C, plus or minus 1º C. After the purified water soak is emptied, the cortical bone sample part was ultrasonically soaked in an alcohol solution SDA-3C (70% ETOHIIPA) at 34º C, plus or minus 1º C for 30 minutes to 2 hours, preferably 1 hour, drained and followed by another ultrasonic soak in USP purified water at 34º C, plus or minus 1º C for 5 minutes. The purified water soak was emptied and repeated a total of 2 soaks to remove the alcohol and the sample part removed. The sample part was then measured, swabbed and packaged. The package containing the sample undergoes lyophilization or freezing and then undergoes quality assurance.

The principles, preferred embodiments and modes of operation of the present invention have been described in the foregoing specification. However, the invention should not be construed as limited to the particular embodiments which have been described above. Instead, the embodiments described here should be regarded as illustrative rather than restrictive. Variations and changes may be made by others without departing from the scope ofthe present inventions defined by the following claims:

## Claims

**1.** A method for producing a cleaned sterile bone graft suitable for transplantation into a human, comprising the steps of:
a) sonicating a bone graft in a nonionic detergent in an ultrasonic cleaner at a temperature ranging from about 31º C to about 35º C and for a time period ranging from 15 minutes to 2 hours effective to produce a cleaned bone graft essentially free from bone marrow;
b) sonicating said bone graft in a purified water in an ultrasonic cleaner at a temperature ranging from about 33º C to about 37º C a plurality oftimes to remove the nonionic detergent;
c) sonicating said bone graft in hydrogen peroxide ranging in concentration from about 1% to about 30% in an ultrasonic cleaner at a temperature ranging from about 33º C to about 37º C for a time period ranging from 10 minutes to about 2 hours effective to retain osteoinductivity;
d) sonicating said bone graft in a purified water in an ultrasonic cleaner at a temperature ranging from about 33º C to about 37º C a plurality of times to remove the hydrogen peroxide; and
e) sonicating saidbone graft in an alcohol solution at a temperature ranging from 33º C to 37º C for 30 minutes to 2 hours effective to produce a clean sterile bone graft.

**2.** The method of claim 1, wherein said bone graft is taken from a group consisting of cortical bone, cancellous bone, cortical cancellous bone and soft tissue.

**3.** The method of claim 1 including a further step (f) sonicating a bone graft in purified water in an ultrasonic cleaner at a temperature from about 33º C to about 37º C a plurality of times to remove the alcohol from the bone graft.

**4.** The method of claim 1 wherein said nonionic detergent comprises a detergent selected from the group consisting of: N, N-Dimethyldodecylamino-N-oxide, Octylglucoside, Polyoxyethylene (PEG) alcohols, Polyoxyethylene-p-t-octylphenol, Polyoxyethylene nonylphenol, Polyoxyethylene sorbitol esters, Polyoxy-propylene-polyoxyethylene esters, p-isoOctylpolyoxy-ethylene-phenol formaldehyde polymer.

**5.** The method of claim 1 wherein said alcohol solution comprises an aqueous combination of ethanol ranging from 40% to 95% and isopropyl alcohol ranging between 0% to 10%.

**6.** The method of claim 1 wherein said viruses are taken from a group consisting of Bovine Viral Diarrhea, Human Immunodeficiency Virus, Pseudorabies, Hepatitis A, Polio and Porcine Parvovirus are cleared from the bone graft at least two logs.

**7.** The method of claim 6 wherein the viruses Bovine Viral Diarrhea, Human Immunodeficiency Virus and Polio are cleaned from the bone graft at least 10 logs.

**8.** The method of claim 6 wherein the Human Immunodeficiency Virus is reduced over a quadrillion times (10¹⁵).

**9.** A bone graft suitable for transplantation into a human produced by the process as claimed in claim 1 wherein said bacteria are taken from a group consisting of Candida albicans, Staphylococcus aureus, Staphylococcus epidermidis, Escherichia coli, Pseudomonas aeruginosa, and Bacillus subtilis are reduced at least ten logs.

**10.** The method of claim 1 wherein the osteoinductivity score ofthe bone graft after step (c) ranges from about 3.50 to about 2.80.

**11.** A method for producing a cleaned, sterile bone graft with a viral clearance of at least two logs suitable for transplantation into a human, comprising the steps of:
a) sonicating a bone graft with a nonionic detergent in an ultrasonic cleaner at a temperature and for a time period effective to produce a cleaned bone graft essentially free from bone marrow;
b) sonicating said bone graft in purified water in an ultrasonic cleaner at a temperature and for a time period effective to remove the detergent;
c) sonicating said bone graft in hydrogen peroxide solution from about 33º C to about 37º C for a time period effective to produce a bone graft with an osteoinductivity score ranging from 2.0 to 3.8;
d) sonicating said bone graft in purified water in an ultrasonic cleaner at a temperature and for a time period effective to remove the hydrogen peroxide;
e) sonicating said bone graft in an alcohol solution at a temperature and for a time effective to sterilize bacteria at least five logs; and
f) sonicating said bone graft inpurified water in an ultrasonic cleaner at a temperature and for a time period effective to remove the alcohol.

**12.** The method of claim 11 wherein said nonionic detergent comprises a detergent selected from the group consisting of:: N, N-Dimethyldodecylamino N-oxide, Octylglucoside, Polyoxyethylene (PEG) alcohols, Polyoxyethylene-p-t-octylphenol, Polyoxyethylene nonylphenol, Polyoxyethylene sorbitol esters, Polyoxy-propylene-polyoxyethylene esters, p-isoOctylpolyoxy-ethylene-phenol formaldehyde polymer.

**13.** The method of claim 11 wherein said alcohol comprises one or more members selected from the group consisting of ethanol, isopropanol, and mixtures thereof

**14.** The method of claim 11 wherein viruses taken from a group consisting of Bovine Viral Diarrhea, Hepatitis C, Human Immunodeficiency Virus, CMV/Herpes, Pseudorabies, Hepatitis A, Polio/Picomaviridae and Human Parvovirus B19 are cleared from the bone graft.

**15.** The method of claim 14 wherein the Human Immunodeficiency Virus is reduced over a quadrillion times (10¹⁵).

**15.** A bone graft suitable for transplantation into a human produced by the process as claimed in claim 11 whereby the bone graft has an osteoinductivity score over 2.0 and viral clearance of at least two logs.

**16.** The method of claim 11 wherein said bacteria are taken from a group consisting of Candida albicans, Staphylococcus aureus, Staphylococcus epidermidis, Escherichia coli, Pseudomonas aeruginosa, and Bacillus subtilis.

**17.** A method for producing a cleaned bone graft suitable for transplantation into a human, comprising the steps of:
a) sonicating a bone graft in a nonionic detergent in an ultrasonic cleaner at an energy level ranging from between 10 - 180 W/sq. in. at a temperature from about 33º C to about 35º C and for a time period ranging from 15 minute to 1 hour to provide a cleaned bone graft essentially free from bone marrow;
b) sonicating said bone graft in purified water in an ultrasonic cleaner at an energy level ranging from between 10 - 180 W/sq. in. at a temperature from about 33º C to about 35º C and for a time period effective to remove the detergent;
c) sonicating said bone graft in a solution of hydrogen peroxide ranging in strength from about 1.5% to about 30% at an energy level ranging from between 10 - 180 W/sq in. at a temperature above 33º C to about 35º C for 15 minutes to 3 hours to produce a bone graft having an osteoinductivity score of at least 2.0;
d) sonicating said bone graft in purified water in an ultrasonic cleaner at an energy level ranging from 10 - 180 W/sq. in. at a temperature ranging from about 33º C to about 35º C and for a time period effective to remove the hydrogen peroxide;
e) sonicating said bone graft in an alcohol in an ultrasonic cleaner at an energy level ranging from 10 - 180 W/sq. in. at a temperature ranging from about 33º C to about 35º C for a time period effective to reduce organisms at least two logs; and
f) sonicating said bone graft in purified water in an ultrasonic cleaner at an energy level ranging from 10 - 180 W/sq. in. at a temperature ranging from about 33º C to about 35º C for a time period effective to remove the alcohol.

**18.** A method for producing a cleaned pre-shaped bone graft suitable for transplantation into a human, comprising the steps of:
a) sonicating a bone graft with a nonionic detergent in an ultrasonic cleaner with a pressurized rinse at a temperature and for a time period effective to produce a substantially cleaned bone graft;
b) cutting the bone graft into an appropriate shape or shapes;
c) sonicating a bone graft in a nonionic detergent in an ultrasonic cleaner at a temperature and for a time period ranging from 15 minute to 1 hour to produce a cleaned bone graft essentially free from bone marrow;
d) sonicating said bone graft in purified water in an ultrasonic cleaner at a temperature and for a time period effective to remove the detergent;
e) sonicating said bone graft in a solution of hydrogen peroxide ranging from about 1.5% to about 30% in an ultrasonic cleaner at a temperature and for a time period effective to produce a bone graft to reduce viruses at least two logs resulting in a bone graft having an osteoinductivity score of at least 2.0;
f) sonicating said bone graft in purified water in an ultrasonic cleaner at a temperature and for a time period effective to remove the hydrogen peroxide; and
g) sonicating said bone graft with an alcohol at a temperature and for a time effective to reduce bacteria and clear viruses from said bone graft at least two logs.

**19.** The method of claim 18 wherein said alcohol comprises one or more members selected from the group consisting of ethanol, isopropanol, and mixtures thereof
